# EUROPEAN PATENT APPLICATION

(11) **EP 2 305 190 A1**
(43) Date of publication of application: **06.04.2011**
(21) Application number: 09172157.1
(22) Date of filing: 03.10.2009
(51) Int. Cl.: A61F 9/02

(54) **Injection moulded glasses and method of their dispensing**

(71) Applicant: Quickex AB, 211 38 Malmö (SE)
(72) Inventor: Rabe, Mats, 211 38, Malmo (SE); Molander, Henrik, 211 38, Malmo (SE)
(74) Representative: Psiorz, Grzegorz

(57) **Abstract**

The object of the invention are safety glasses and the safety glasses dispensing method.

Safety glasses (G) made by method of injection molding from transparent plastic material, characterized by the fact that they constitute an ergonomically formed element, wherein the side bows (1) are separated from the central one (2) with the dents (3) facilitating the appropriate bending to working position. Preferably the plastic used for molding belongs to thermoplastic especialy polycarbonates. Another important factor is the method of dispensing the safety glasses (G) using the dispenser (5) of the shape similar to rectangular, containing disposable safety glasses (G) placed in the vicinity of devices or the premises, which require application of eye protection measures, and users before proceeding with the work in the risk conditions collect the necessary glasses, adjust them to the shape of their head by proper folding, and dispose them after work. Unfloding the bows (1) and (2) while taking the glasses off leads to destruction of the glasses.

## Description

The object of the invention are injection moulded glasses and method of their dispensing. In many work places usage of safety glasses is required in order to protect worker's eyes from damage by a variety of biological factors, chemicals or particles such as dust, filings or chips. The same situation there is on the beach or ice glacier, for example. The people need to protect their eyes against sunshine.

Providing this type of eye protection to employees performing work continuously in conditions of risk is not problematic. The problem however arises when work is preformed in risk conditions on occasional basis. Worker, before preforming work in the risk conditions, should supplement equipment with the safety glasses, unrequired in a permanent place of work. This additional burden and the fact that the work will be performed under risk conditions only for a short period of time leads to the situation in which most workers are willing to compromise their safety and take the risk of working without the protection of their sight. Placing general use safety glasses in the area of devices, mostly for hygiene reasons is not the best option.

British patent application GB 2442755 present a container for dispensing new safety spectacles. Container is usually placed at the entrance to the premises in which the goggles should be used or close to the risky devices in a manner that makes collection of the glasses possible for a worker. The disadvantage of this type of solution is an increased costs incurred from the purchase of this type of glasses or goggles.

Another attempt to solve the problem is presented in the Swedish patent application SE 0602365 This solution uses a simple press which cuts off disposable glasses - when required - from a sheet- or foil material of transparent plastic. These safety glasses utilize a spring action which is sufficient for fixing and retaining the safety glasses on a wearer's head.

The essence of the invention are injection moulded glasses made from transparent plastic material, characterized by the fact that they constitute an ergonomically formed element, yet the side bows are separated from the central one with the dents facilitating the appropriate bending to working position. It is important that the plastic used for molding belonged to thermoplastic, especialy polycarbonates or polymethacrylates. Another important factor is the method of dispensing the safety glasses through the dispenser of the shape similar to rectangular, containing disposable safety glasses, placed in the vicinity of devices or the premises, which require application of eye protection measures, and users before proceeding with the work in the risk conditions would collect the necessary glasses, adjust them to the shape of their head by proper folding, and dispose them after work. It is also significant that unfloding the bows while taking the glasses off would lead to destruction of the glasses.

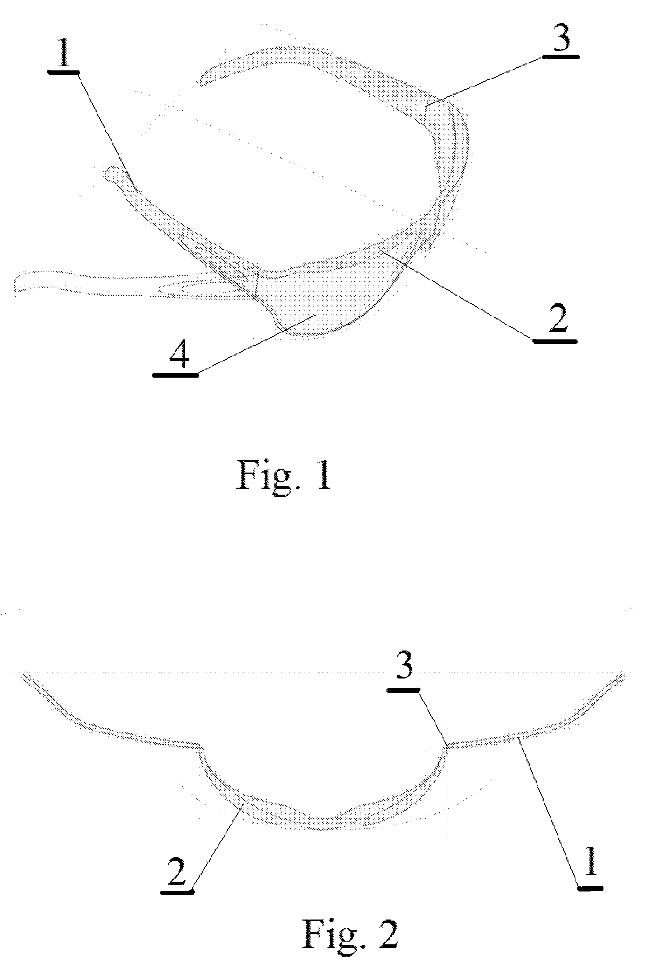

The subject of the invention is shown in the drawing, in which Fig. 1 shows the view of safety glasses, and Fig. 2 shows view from the top on the glasses right after taking them out of the form. Fig. 3 shows the view of the version of the dispenser, and Fig. 4 presents views from the front and side of the dispenser and the longitudinal cross-section showing the glasses position in the dispenser. Fig. 5 shows another variant of the implementation of the dispenser, and Fig 6 the arrangement of glasses inside the distributor of this release.

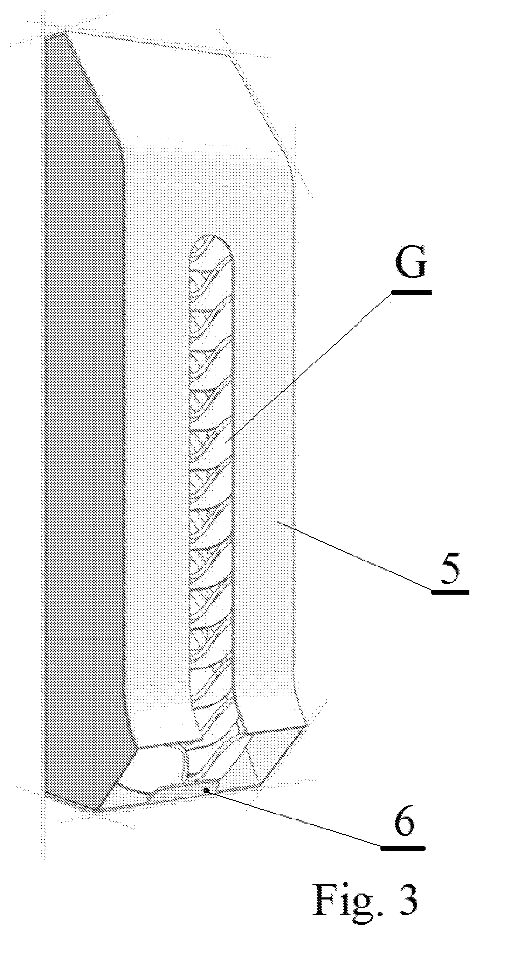

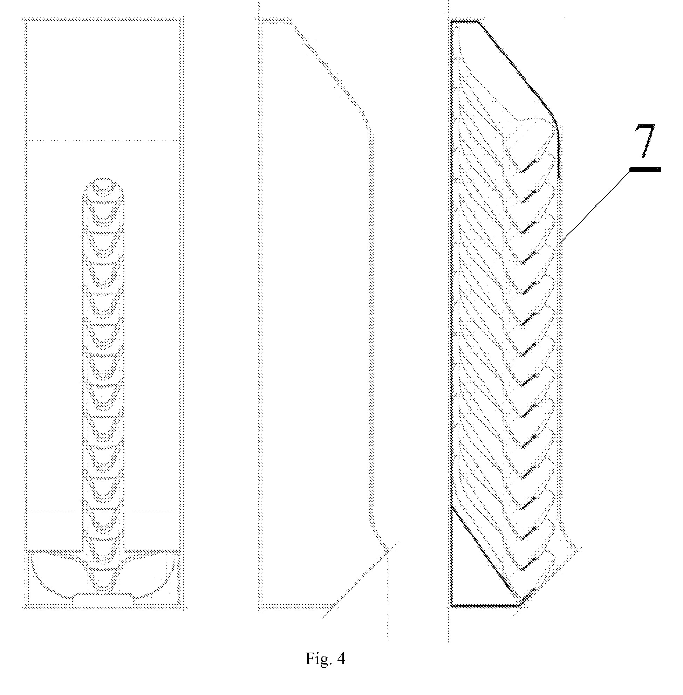

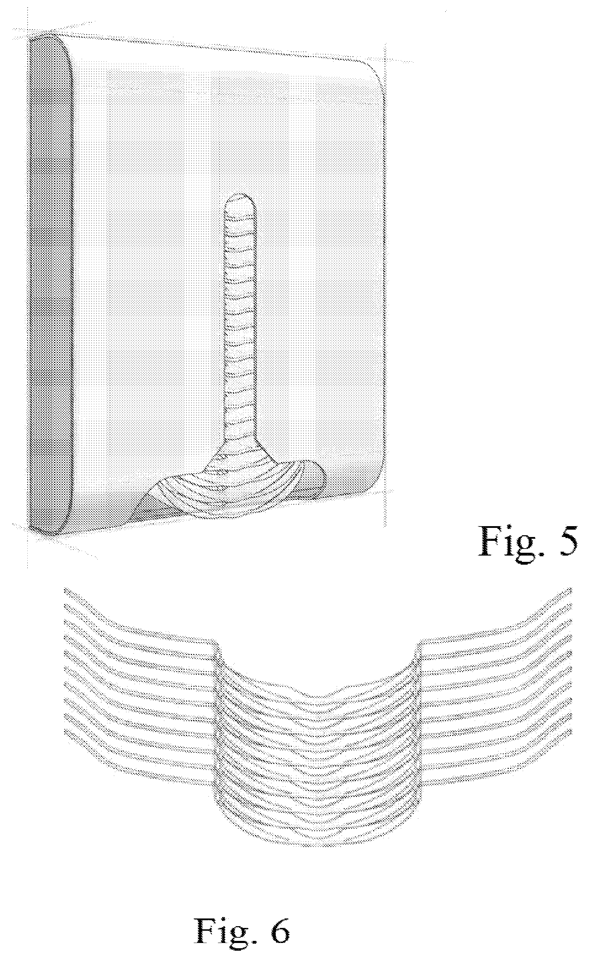

Glasses G are made out of polycarbonate in the form of one component obtained by injection molding. In the process of injection molding glasses are formed in such way that from the top perspective they resemble three bows: front 2 and two sides -1 separated from each other by bendable dents 3 which are used to fit the glasses to the wearer's head shape. Side bows 1 are the sides of glasses formed as an ear piece which can be attached to wearer's ears, and front bow 2 is the front of the glasses G equipped with eye safety protection glasses 4. Glasses G are placed prior the use in dispenser 5 of approximately rectangular shape, in a way that the protrusions of the front bow 2 are directed downwards. Depending on the version they can be initially folded, as shown in Fig.3 and 4 or unfolded, in a way they had been created in the injection molding form. This second version of the dispenser was shown in Fig. 5, and the position of the glasses in the dispenser is shown in Fig. 6. The dispenser 5 is equipped with slit 7 which enables monitoring the state of its load. The bottom part contains the plate 6 placed to secure sunglasses G from falling out of the dispenser 5. Dispenser 5 loaded with disposable protective glasses is placed in the vicinity of devices or the premises, which require application of eye protection measures, and users before proceeding with the work in the risk conditions collect the necessary glasses, adjust them to the shape of their head by proper folding, and dispose them after work. Unfloding the bows while taking the glasses off leads to destruction of the glasses.

## Claims

**1.** Injection moulding glasses made from transparent plastic **characterized in that** they make one ergonomically formated element, wherein side bows (1) are parted from the central one (2) by the dents (3) facilitating the appropriate bending to working position.

**2.** Injection moulding glasses according to claim 1 **characterized in that** the plastic used for molding belongs to polycarbonates.

**3.** Injection moulding glasses according to claim 1 **characterized in that** the plastic used for molding belongs to thermoplastic.

**5.** Injection moulding glasses according to claim 3 **characterized in that** the plastic used for molding belongs to polycarbonates.

**6.** Injection moulding glasses according to claim 3 **characterized in that** the plastic used for molding belongs to polymethacrylates.

**7.** Method of dispensing safety glasses according to claim 1 **characterized in that** the dispenser (5) of the shape similar to rectangular, containing disposable safety glasses (G) is placed in the vicinity of devices or the premises, which require application of eye protection measures, and users before proceeding with the work in the risk conditions collect the necessary glasses, adjust them to the shape of their head by proper folding, and dispose them after work.

**8.** Method according to the claim 7 **characterized in that** the unfoding the bows (1) and (2) while taking the glasses off leads to destruction of the glasses.
